# EUROPEAN PATENT SPECIFICATION

(11) **EP 2 210 609 B1**
(45) Date of publication and mention of the grant of the patent: **23.10.2013**
(21) Application number: 08845900.3
(22) Date of filing: 28.10.2008
(51) Int. Cl.: A61K 35/74, A23K 1/16, A23L 1/30, A61P 3/04, A61P 3/06, A61P 9/12, A23L 2/52

(54) **AGENT FOR PROMOTING THE SECRETION OF ADIPONECTIN AND/OR INHIBITING THE DECREASE IN THE SECRETION OF ADIPONECTIN**
MITTEL ZUR FÖRDERUNG DER SEKRETION VON ADIPONECTIN UND/ODER ZUR HEMMUNG DER ABNAHME DER ADIPONECTIN-SEKRETION
AGENT FAVORISANT LA SÉCRÉTION D'ADIPONECTINE ET/OU INHIBANT LA DIMINUTION DE LA SÉCRÉTION D'ADIPONECTINE

(30) Priority: 29.10.2007 JP 2007280195
(43) Date of publication of application: 28.07.2010
(73) Proprietor: Megmilk Snow Brand Co., Ltd., Sapporo-shi Hokkaido 0650043 (JP)
(72) Inventor: KUNIEDA, Yukiko, Tokyo 114-0023 (JP); SETO, Yasuyuki, Kawagoe-shi Saitama 350-1165 (JP); KADOOKA, Yukio, Kawagoe-shi Saitama 350-1165 (JP)
(74) Representative: Wakerley, Helen Rachael
(86) International application number: PCT/JP2008/069568
(87) International publication number: WO 2009/057604

(56) References cited:
- EP-A1- 1 260 227
- WO-A1-2007/029631
- WO-A1-2007/043933
- WO-A1-2008/029505
- WO-A1-2008/029505
- JP-A- 1 268 641
- JP-A- 2003 306 436
- JP-A- 2006 306 866
- JP-A- 2007 153 741
- DATABASE WPI Week 200746 Thomson Scientific, London, GB; AN 2007-472892 XP002625759, & JP 2007 153741 A (SNOW BRAND MILK PROD CO LTD) 21 June 2007 (2007-06-21)
- DATABASE WPI Week 200851 Thomson Scientific, London, GB; AN 2008-J01385 XP002625760, & JP 2008 156299 A (KAO CORP) 10 July 2008 (2008-07-10)

## Description

### Technical Field

The present invention relates to an agent that promotes the secretion of and/or suppresses decrease of adiponectin containing culture supernatant of *Lactobacillus gasseri* SBT2055 (FERM BP-10953) as an active ingredient, a novel food or beverage product having the effect of promoting the secretion of and/or suppressing decrease of adiponectin, and a novel feed having the effect of promoting the secretion of and/or suppressing decrease of adiponectin. Taking the agent for promoting the secretion of and/or suppressing decrease of adiponectin of the present disclosure can promote the secretion of and/or suppress decrease of adiponectin from fat tissues. The decrease in the secretion of adiponectin is believed to cause/aggravate the metabolic syndrome in which multiple conditions such as hypertension, hyperlipidemia and diabetes occur concurrently, and the compositions described herein are useful for its prevention and treatment.

### Background Art

Hypertrophy of the fat cells and excessive accumulation of the visceral fat may cause the concurrent occurrence of multiple disease conditions such as hypertension, hyperlipidemia and diabetes. These disease conditions are collectively called the metabolic syndrome, which is becoming in the recent years an important national health issue for which countermeasures are urgently needed. Visceral adipose tissues secrete endocrine factors such as adiponectin, plasminogen activator inhibitor, tumor necrosis factor (TNF-α) and leptin, and contribute to the maintenance of homeostasis in the body. However, hypertrophy of the fat cells causes abnormality in the secretion of these factors, resulting in excessive or deficient secretion. It is becoming clear from recent studies that this loss of secretory balance is deeply involved in the onset and aggravation of the metabolic syndrome. Abnormality in the adiponectin secretion, in particular, is believed to have the most profound influence (see, for example, Non-Patent Document 1).

Adiponectin is a molecule consisting of 244 amino acids, and is secreted from the adipose tissues. Adiponectin has been shown to have the effect of improving insulin resistance, as well as the effect of promoting fat burning in the liver and muscles. It has also been found that adiponectin promotes the uptake of glucose and fatty acids from the bloodstream into cells. Accumulation of fat in muscles or in the liver or other organs results in inefficient sugar uptake and may lead to diabetes. Normally, however, adiponectin appears to facilitate the maintenance of the nutritional balance in the body by breaking down the fat and sugars that have temporarily become excessive. It is believed that the adiponectin-secreting fat cells become less active as the obesity progresses, and this leads to the loss of the nutritional balance in the body. Thus, it is expected that a wide range of metabolic syndrome conditions, such as hypertension, abnormal lipid metabolism and diabetes, can be improved simultaneously by normalizing the adiponectin secretion.

Drugs and artificial compounds that can increase adiponectin levels have been sought after, but in light of the risk of the potential side effects associated with these drugs and artificial compounds, much attention has been directed towards the studies of the food components that may, at minimum, slow down the advancement of the diseases through the daily diet. A number of plant extracts have been disclosed, such as apple extracts (for example, see Patent Document 1), hop bract extracts (for example, see Patent Document 2), green tea catechin (for example, see Patent Document 3), rice bran extracts (for example, see Patent Document 4), and turmeric bulb extracts (for example, see Patent Document 5), as substances that may enhance adiponectin concentration in the blood. However, whether they are practical as a material for making pharmaceutical or food/beverage products is questionable, because they may require complicated extraction conditions, use rarely available raw materials for extraction, or have undesirable effects on the flavor of the food to which they are added.

On the other hand, the food products made by the use of lactic acid bacteria fermentation are diverse (examples include cheeses, yogurts and pickles), may be provided relatively cheaply, and have been produced worldwide in large amounts throughout the history because of their captivating tastes. Lactic acid bacteria, by means of fermentation, produce numerous decomposition products and metabolites, among which a number of functional health food components are being identified, but it is believed that the functions of many of them still remain to be understood. The present inventors discovered that the peptides derived from the milk proteins isolated from the cheeses that had been aged with lactic acid bacteria had the effects of promoting adiponectin production (for example, see Patent Document 6).

*Lactobacillus gasseri* is known to provide the effects against pathogenic infections (for example, see Patent Document 7), effects of preventing inflammatory bowel disease and irritable bowel syndrome (for example, see Patent Document 8), effects of inhibiting bone resorption (for example, see Patent Document 9), effects of enhancing the immune system (for example, see Patent Document 10), as well as the effects of preventing diabetes-associated complications (for example, see Patent Document 11) and the effects of inhibiting the increase of serum cholesterol levels (for example, see Patent Document 12). JP 2007 153741A teaches the use of bacteria harvested from a culture and/or microbial cell of *Lactobacillus gasseri* SBT 20055 as an inhibitor for accumulation of fat surrounding kidneys and for treating metabolic syndrome. However, it has never been known that a culture supernatant (i.e. the liquid component that remains after the milk protein-precipitates and the bacterial cell components have been removed from a bacterial culture) of *Lactobacillus gasseri* or any other type of lactic acid bacterium has, on its own, the effect of increasing the adiponectin level.

The milk protein-precipitates and the bacterial cell components that have been coagulated in the fermentation process greatly affect the flavors of the dairy products, and may sometimes impair the quality of the products and damage their commercial values. The technologies for removing the precipitates from the milk fermentation mixture are also being developed (for example, see Patent Document 13), and the culture supernatants from the milk fermentation that could provide desirable flavors and captivating tastes for the food products offer extremely high industrial applicability.
Patent Document 1: JP 2006-193502 A; Patent Document 2: JP 2006-193501 A
Patent Document 3: JP 2006-131512 A; Patent Document 4: JP 2005-68132 A
Patent Document 5: JP 2005-60308 A; Patent Document 6: JP 2007-254448 A
Patent Document 7: JP H08-268899 A; Patent Document 8: JP 2003-95963 A
Patent Document 9: JP 2004-315477 A; Patent Document 10: JP 2006-69993 A
Patent Document 11: JP 2003-252770 A; Patent Document 12: JP 2003-306436 A
Patent Document 13: JP H06-319447 A;
Non-Patent Document 1: J. Clin. Invest., 116:1784-1792

### Summary of Invention

### Problems to be solved by Invention

The objective of the present invention is to provide a lactic acid bacteria culture supernatant that has excellent applicability and general usefulness as a food product material and at the same time is effective in the prevention and treatment of the metabolic syndrome due to its effect of promoting the secretion of adiponectin in the body.

### Means of solving the problem

The present inventors have carried out extensive studies for achieving the above objective, and as a result, discovered that the culture supernatant of *Lactobacillus gasseri* SBT2055 (FERM BP-10953) shows extremely strong effects of promoting the secretion of and/or suppressing decrease of adiponectin, which has led to the completion of the present invention.

Thus, the present invention provides a composition comprising a culture supernatant of *Lactobacillus gasseri* SBT2055 (FERM BP-10953) as an active ingredient for use in the treatment or prevention of hypertension, hyperlipidemia, diabetes mellitus type-2, abdominal obesity, dyslipidemia, insulin resistance or metabolic syndrome.
The composition of the present invention may be in the form of a medicament, a food or beverage product or an animal feed.

### The effects of Invention

The compositions according to the present invention are effective in the prevention and treatment of the metabolic syndrome that is believed to be caused by the decrease in the adiponectin levels in the blood. Also, since the compositions according to the present invention use the culture supernatant of *Lactobacillus gasseri* SBT2055 (FERM BP-10953), they are characterized by excellent applicability and general usefulness as highly pure materials for food products, can be provided relatively cheaply in large quantities, and are also noted for being highly safe.

### Brief Description of Drawings

[Figure 1]
   This figure shows the results of the measurements of the adiponectin concentrations. (Test Example 1)

### Description of Embodiments

The present inventors had searched, among many lactic acid bacteria derived from fermented milk or from the human body, for the bacterial strains that could provide the effects of ameliorating diabetes or lowering blood cholesterol levels and also provide suitable flavors and textures in the food product applications. The search was continued with an additional criterion, i.e. the target bacterial strains were required to produce culture supernatants that exhibited the effects of promoting the secretion of and/or suppressing decrease of adiponectin. From this search, the *Lactobacillus gasseri* SBT2055 strain has been selected. This bacterial strain has been deposited to the National Institute of Advanced Industrial Science and Technology Patent Microorganism Depositary under the deposition number of FERM BP-10953.

It was never known that the culture supernatant of *Lactobacillus gasseri* SBT2055 (FERM BP-10953), from which the bacterial cell components had been removed, had on its own the effect of promoting the secretion of and/or suppressing decrease in adiponectin, and this fact has been revealed for the first time by the present inventors.

Next, the method of culturing these lactic acid bacteria will be described. Various culture media, such as milk media, media containing milk components, and semi-synthetic media not containing the milk components, may be used, but the media containing whey powder as a supplementary milk component are especially preferable. The bacteria are usually cultured in the stationary culture or the neutral culture in which the pH is maintained within a certain range, but any culturing methods and conditions that are favorable for the bacterial growth may be used. The culture supernatant can be obtained by removing the milk protein precipitates and the bacterial cell components from the bacterial culture described above, by means of appropriate methods such as centrifugation and filtration.

The culture supernatant obtained above constitutes an active ingredient in the compositions according to the present invention. The supernatant may be used directly, or dried into powder before it is used as the active ingredient. Any drying methods may be used, but freeze-drying is preferable because it can suppress the degradation of the components. The powder may be mixed with appropriate excipients such as lactose and formulated into various forms of medications, such as powder medications, tablets, pills, capsules, and syrups. These medications are preferably administered through the oral route.

The present invention may be in the form of a food or beverage product. This contains the culture supernatant obtained above as an active ingredient and provides the effect of promoting the secretion of and/or suppressing decrease of adiponectin. The food or beverage product may be in any form; it may be the culture supernatant itself, or a food or beverage product that contains the said culture supernatant. The culture supernatant or the dried product thereof may be added to any other food or beverage items when they are eaten or drunk, added to the food or beverage products when they are manufactured, or admixed with their preparation or production materials. Examples of the food and beverage products include dairy drinks, fermented milks, fruit drinks, jellies, candies, egg products such as mayonnaise, confectioneries such as butter cakes, and breads. The examples also include various powdered milks and other nutritional compositions aimed at babies and low birth weight infants.

The present invention may be in the form of an animal feed. This contains the culture supernatant obtained above as an active ingredient and provides the effect of promoting the secretion of and/or suppressing decrease of adiponectin. Similarly to the food and beverage products described above, the animal feed to which the culture supernatant is added may be in any form, and the culture supernatant or the dried product thereof may be added to the materials during the manufacturing process of the feed.

The administered dose or the admixed amount of the *Lactobacillus gasseri* SBT2055 (FERM BP-10953) culture supernatant, for a typical adult, may be adjusted to 10 to 200 g per day, or 0.5 to 50 g of the dried product thereof per day, in order to exert the effect of promoting the secretion of and/or suppressing decrease of adiponectin.

Below, the Examples and the Test Example will be shown to explain the present invention in further details, but they will be provided only as examples, and the scope of the present invention is not limited by these examples.

### Example 1

### (Preparation of the culture supernatant 1)

The reduced whey culture medium (containing 13 weight % whey powder and 0.5 weight % yeast extract) was sterilized at 95°C for 30 minutes, and then inoculated with *Lactobacillus gasseri* SBT2055(FERM BP-10953). The culture was incubated at 37°C for 16 hours, and then centrifuged at 3,500 rpm for 20 minutes to produce the culture supernatant that was clear of the precipitated/sedimented materials. This supernatant may be used directly as an agent for promoting the secretion of and/or suppressing decrease of adiponectin according to the present disclosure.

### Example 2

### (Preparation of the culture supernatant 2)

Reduced non-fat milk medium (containing 13 weight % non-fat dried milk and 0.5 weight % yeast extract) was sterilized at 95°C for 30 minutes and then inoculated with *Lactobacillus gasseri* SBT2055(FERM BP-10953). The culture was incubated at 37°C for 16 hours, and then centrifuged at 3,500 rpm for 20 minutes to produce the culture supernatant that was clear of the precipitated/sedimented materials. This supernatant may be used directly as an agent for promoting the secretion of and/or suppressing decrease of adiponectin according to the present disclosure.

### [Test Example 1]

### (Test administration to the fat cells)

In this test, the culture supernatant obtained in Example 1 was administered to the primary culture visceral fat cells. The test was conducted by using the rat primary culture visceral fat cells (VAC01, Cell Garage corporation) and the visceral fat cell differentiation induction medium (Cell Garage corporation). The cells that had been stored frozen were thawed according to the protocol of the Cell Garage corporation and seeded in the 24-well plates. The day of the seeding was designated as the day 0, and on the day 5 in which the secretion of adiponectin became active, the culture supernatant obtained from the bacterial culture in the reduced whey medium was added to the fat cell differentiation induction medium. In a comparison experiment, no bacterial culture supernatant was added to the fat cell differentiation induction medium. The cells were cultured for 2 hours at 37°C under 0.5% carbon dioxide partial pressure, and the culture medium was harvested. The concentration of the adiponectin secreted into the medium was determined by using the adiponectin ELISA kit (Otsuka Pharmaceutical corporation). The measurement values were normalized by the amounts of the DNA extracted from the corresponding wells.

### (The results of the test administration to the fat cells)

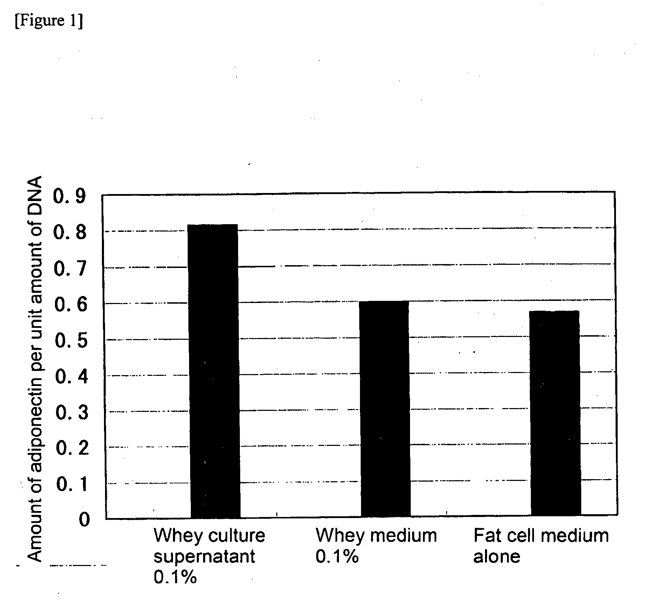

Figure 1 shows the adiponectin concentration measurement values obtained from the test in which the reduced whey medium had been used for culturing *Lactobacillus gasseri.* The amount of the secreted adiponectin was increased by approximately 1.44 fold when the *Lactobacillus gasseri* culture supernatant was administered to the fat cells, relative to the cells to which no bacterial culture supernatant was given. The amount of the secreted adiponectin was 1.36 fold higher when compared to the cells to which the reduced whey medium alone was added.

The results above have indicated that the culture supernatant of *Lactobacillus gasseri* has the effect of strongly promoting the secretion of adiponectin in the fat cells, and that this effect is attributed to the factors derived from *Lactobacillus gasseri* SBT2055 (FERM BP-10953) and not to the reduced whey medium alone.

### Example 3

### [Production of the tablets]

The reduced whey culture medium (containing 13 weight % whey powder and 0.5 weight % yeast extract) was sterilized at 95°C for 30 minutes, and then inoculated with *Lactobacillus gasseri* SBT2055(FERM BP-10953). The culture was incubated at 37°C for 16 hours, and then centrifuged at 3,500 rpm for 20 minutes to produce the culture supernatant that was clear of the precipitated/sedimented materials. This was subjected to the freeze-drying treatment to produce the culture supernatant powder. One part of the culture supernatant powder was mixed with four parts of non fat dried milk, and this powder mix was processed in the tablet press machine (1 g per tablet) according to the conventional procedure, to produce the tablets of the present invention that contained 200 mg of the *Lactobacillus gasseri* culture supernatant for promoting the secretion of and/or suppressing decrease of adiponectin.

### Example 4

### (Production of the powder agent)

After 5 liters of the reduced whey medium was inoculated with *Lactobacillus gasseri* SBT2055(FERM BP-10953), the stationary culture was incubated at 37°C for 18 hours. When the culturing step was completed, the culture was centrifuged at 7,000 rpm for 15 minutes to produce the culture supernatant that was clear of the precipitated/sedimented materials. Next, this culture supernatant was mixed with an equal volume of the dispersion medium containing 10 weight % non fat dried milk and 1 weight % sodium glutamate. After the pH was adjusted to 7, the mixture was freeze-dried. The resultant freeze-dried material was granulated through a "60 mesh" sieve to produce the freeze-dried culture supernatant product. In accordance with the "Powder Agent" section in the 13th Revised Edition Japanese Pharmacopoeia Booklet Pharmaceutical Processing Rules, 400 g lactose (Nikkyoku) and 600 g potato starch (Nikkyoku) were added to 1 g freeze-dried culture supernatant. They were mixed homogeneously to produce the agent for promoting the secretion of and/or suppressing decrease of adiponectin of the present disclosure.

### Example 5

### (Production of the capsules)

After 5 liters of the reduced whey medium was inoculated with *Lactobacillus gasseri* SBT2055(FERM BP-10953), the stationary culture was incubated at 37°C for 18 hours. When the culturing step was completed, the culture was centrifuged at 7,000 rpm for 15 minutes to produce the culture supernatant that was clear of the precipitated/sedimented materials. Next, this culture supernatant was mixed with an equal volume of the dispersion medium containing 10 weight % non fat dried milk and 1 weight % sodium glutamate. After the pH was adjusted to 7, the mixture was freeze-dried. The resultant freeze-dried material was granulated through a "60 mesh" sieve to produce the freeze-dried culture supernatant product. The ingredients were mixed according to Table 1, granulated, and encapsulated, to produce the capsules for promoting the secretion of and/or suppressing decrease of adiponectin of the present disclosure.

**[Table 1]**

| | | |
|---|---|---|
| Freeze-dried culture supernatant | | 20.0 (weight %) |
| Lactose | | 24.5 |
| Soluble starch | 55.0 | |
| Magnesium stearate | 0.5 | |

### Example 6

### (Production of the stick-type health food)

After 5 liters of the MRS liquid medium (Difco corporation) was inoculated with *Lactobacillus gasseri* SBT2055(FERM BP-10953), the stationary culture was incubated at 37°C for 18 hours. When the culturing step was completed, the culture was centrifuged at 7,000 rpm for 15 minutes to produce the culture supernatant that was clear of the precipitated/sedimented materials. Next, this culture supernatant was mixed with an equal volume of the dispersion medium containing 10 weight % non fat dried milk and 1 weight % sodium glutamate. After the pH was adjusted to 7, the mixture was freeze-dried. The resultant freeze-dried material was granulated through a "60 mesh" sieve to produce the freeze-dried culture supernatant product. Thirty grams of this *Lactobacillus gasseri* SBT2055 culture supernatant powder was admixed with 40 g of an equal mixture of vitamin C and citric acid, 100 g of granulated sugar, and 60 g of an equal mixture of corn starch and lactose. The mixture was packed in a stick-shape package to produce the stick-type health food for promoting the secretion of and/or suppressing decrease of adiponectin.

### Example 7

### (Production of the beverage)

After 5 liters of the reduced whey medium was inoculated with *Lactobacillus gasseri* SBT2055(FERM BP-10953), the stationary culture was incubated at 37°C for 18 hours. When the culturing step was completed, the culture was centrifuged at 7,000 rpm for 15 minutes to produce the culture supernatant that was clear of the precipitated/sedimented materials. The ingredients were mixed according to Table 2, packed into a container and heat-sterilized, to produce the beverage product for promoting the secretion of and/or suppressing decrease of adiponectin.

**[Table 2]**

| | |
|---|---|
| Culture supernatant | 2.5 (weight %) |
| Sugar | 7.5 |
| Citric acid | 0.6 |
| Apple juice | 10.0 |
| Water | 79.4 |

### Example 8

### (Production of the yogurt)

*Lactobacillus gasseri* SBT2055(FERM BP-10953) was cultured in the MRS liquid medium (Difco corporation). The culture suspensions in the log-phase growth were each used (1% relative to the volume of the whey medium) to inoculate the 13% reduced whey medium supplemented with 0.5% yeast extract (sterilized at 115°C for 20 minutes) to produce the 'mother culture'. The resultant culture was centrifuged at 3,500 rpm for 20 minutes to produce the culture supernatant that was clear of the precipitated/sedimented materials. This culture supernatant was added at 2.5% to the yogurt mix that had been sterilized at 100°C for 10 minutes and chilled. To this, the starter mix consisting of Lactobacillus bulgaricus and Streptococcus thermophilus was added at 3%, and the mixture was allowed to undergo fermentation at 37 °C. When the lactic acid acidity reached 0.85, the mixture was cooled, the fermentation was terminated, and the yogurt for promoting the secretion of and/or suppressing decrease of adiponectin was thus obtained.

### Example 9

### (Production of the yogurt drink)

Four kilograms of granulated sugar, 3 kg of water, and 0.15 kg of pectin were added to 43 kg of the yogurt obtained in Example 8. These components were mixed to homogeneity to produce 50 kg of the yogurt drink for promoting the secretion of and/or suppressing decrease of adiponectin. This yogurt drink had a mild, desirable flavor, and the pH of 3.6.

### Example 10

### (Production of the dog feed)

After 5 liters of the MRS liquid medium (Difco corporation) was inoculated with *Lactobacillus gasseri* SBT2055(FERM BP-10953), the stationary culture was incubated at 37°C for 18 hours. When the culturing step was completed, the culture was centrifuged at 7,000 rpm for 15 minutes to produce the culture supernatant that was clear of the precipitated/sedimented materials. Next, this culture supernatant was mixed with an equal volume of the dispersion medium containing 10 weight % non fat dried milk and 1 weight % sodium glutamate. After the pH was adjusted to 7, the mixture was freeze-dried. The resultant freeze-dried material was granulated through a "60 mesh" sieve to produce the freeze-dried culture supernatant product. The ingredients were mixed according to Table 3 to produce the dog feed for promoting the secretion of and/or suppressing decrease of adiponectin.

**[Table 3]**

| | |
|---|---|
| Freeze-dried culture supernatant | 2.5 (weight %) |
| Non fat dried milk | 13.5 |
| Soybean meal | 12.0 |
| Soybean oil | 4.0 |
| Corn oil | 2.0 |
| Palm oil | 27.0 |
| Corn starch | 14.0 |
| Wheat flour | 9.0 |
| Wheat bran | 2.0 |
| Vitamin mix | 9.0 |
| Mineral mix | 2.0 |
| Cellulose | 3.0 |

### Industrial Applicability

Taking the composition of the present invention can promote the secretion of and/or suppress the decrease of adiponectin in the adipose tissues. The decrease in the secretion of adiponectin is believed to cause/aggravate the metabolic syndrome in which multiple conditions such as hypertension, hyperlipidemia and diabetes occur concurrently, and the present invention is useful for its prevention and treatment. The culture supernatants from the milk fermentation that can provide desirable flavors and captivating tastes for the food products have extremely high industrial applicability.

| | | |
|---|---|---|
| **0-1** | Form PCT/RO/134 (SAFE) The indications on the deposited microorganism or other biological material (PCT Rule 13bis) were made by: | **JPO-PAS 0352** |
| **0-1-1** | | |
| **0-2** | International Application Number | |
| **0-3** | Applicant's or Agent's Reference Number | **SNOW-164** |
| | | |
| **1** | The indications made below relate to the deposited microorganism or other biological material referred to in the description. | 0001, 0009, 0010, 0011, 0013, 0014, 0019, 0021, 0022, 0025,0026,0027,0028,0030,0031,0033,0035 |
| **1-1** | Paragraph Numbers | |
| **1-3** | Indication of deposit | |
| **1-3-1** | Name of depositary institution | Advanced Industrial Science and Technology International Patent Organism Depositary (IPOD) |
| **1-3-2** | Address of depositary institution | Chuo 6, 1-1-1 Higashi, Tsukuba-City, Ibaraki 305-8566 Japan |
| **1-3-3** | Date of deposit | 26.02.2008 |
| **1-3-4** | Accession Number | **IPOD FERM BP-10953** |
| **1-5** | Designated states for which Indications were made | All designated States |

## Claims

1. A composition comprising a culture supernatant of *Lactobacillus gasseri* SBT2055 (FERM BP-10953) as an active ingredient for use in the treatment or prevention of hypertension, hyperlipidemia, diabetes mellitus type-2, abdominal obesity, dyslipidemia, insulin resistance or metabolic syndrome.

2. A composition according to claim 1 in the form of a medicament.

3. A composition according to claim 1 in the form of a food or beverage product.

4. A composition according to claim 1 in the form of an animal feed.

## Patentansprüche

1. Zusammensetzung, die einen Kulturüberstand von *Lactobacillus gasseri* SBT2055 (FERM BP-10953) als Wirkstoff enthält, zur Verwendung bei der Behandlung oder Verhütung von Hypertonie, Hyperlipidämie, Diabetes mellitus Typ 2, abdominaler Adipositas, Dyslipidämie, Insulinresistenz oder metabolischem Syndrom.

2. Zusammensetzung nach Anspruch 1 in Form eines Medikaments.

3. Zusammensetzung nach Anspruch 1 in Form eines Lebensmittel- oder Getränkeprodukts.

4. Zusammensetzung nach Anspruch 1 in Form eines Futtermittels.

## Revendications

1. Composition comprenant un surnageant de culture de *Lactobacillus gasseri* SBT2055 (FERM BP-10953) en tant que principe actif, à utiliser dans le traitement ou la prévention d'hypertension, d'hyperlipidémie, du diabète sucré de type 2, d'obésité abdominale, de dyslipidémie, d'insulinorésistance ou du syndrome métabolique.

2. Composition selon la revendication 1, sous la forme d'un médicament.

3. Composition selon la revendication 1, sous la forme d'un produit alimentaire ou de boisson.

4. Composition selon la revendication 1, sous la forme d'une nourriture pour animaux.
